(19) Europäisches Patentamt
European Patent Office
Office européen des brevets

(11) Veröffentlichungsnummer: **0 035 708**
**B1**

(12) # EUROPÄISCHE PATENTSCHRIFT

(45) Veröffentlichungstag der Patentschrift: **12.10.83**

(51) Int. Cl.³: **C 07 D 253/06** // C07C103/88, C07C102/08, C07C59/185, A01N43/64

(21) Anmeldenummer: **81101405.9**

(22) Anmeldetag: **26.02.81**

(54) **Verfahren zur Herstellung von 4-Amino-6-tert.-butyl-3-methylthio-1,2,4-triazin-5(4H)-on.**

(30) Priorität: **08.03.80 DE 3008921**

(43) Veröffentlichungstag der Anmeldung: **16.09.81 Patentblatt 81/37**

(45) Bekanntmachung des Hinweises auf die Patenterteilung: **12.10.83 Patentblatt 83/41**

(84) Benannte Vertragsstaaten: **AT BE CH DE FR GB IT LI NL**

(56) Entgegenhaltungen:
**DE-A-2 726 016**
**DE-A-2 732 797**
**DE-A-2 733 180**
**DIE ANGEWANDTE MAKROMOLEKULARE CHEMIE, Band 79, veröffentlicht in 1979, Seiten 193-206 Basel, CH. H.D. SCHARF et al.: «Alpha-Ketoimidgruppierungen als eine der Ursachen für die Lumineszenz thermooxidativ geschädigten Polycaprolactams»**
**CHEMISCHE BERICHTE, Band 97, veröffentlicht in 1964, Seiten 2173-2178 Weinheim, DE. A. DORNOW et al.: «Über 1,2,4-Triazine, I. Darstellung einiger neuer s-Triazolo(3.2-c)-as-triazine»**
**HOUBEN WEYL, «Methoden der organischen Chemie, veröffentlicht in 1952, Seite 709, Band VIII Stuttgart, DE. «Sauerstoffverbindungen III»**

(73) Patentinhaber: **BAYER AG, Zentralbereich Patente, Marken und Lizenzen, D-5090 Leverkusen 1, Bayerwerk (DE)**

(72) Erfinder: **Bonse, Gerhard, Dr., Wolfskaul 3, D-5000 Köln 80 (DE)**
Erfinder: **Blank, Heinz Ulrich, Dr., Am Geusfelde 35, D-5068 Odenthal (DE)**
Erfinder: **Krätzer, Hans, Dr., Am Acker 17, D-5600 Wuppertal (DE)**

**EP 0 035 708 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

Verfahren zur Herstellung von 4-Amino-6-tert.-butyl-3-methylthio-1,2,4-triazin-5(4H)-on

Die vorliegende Erfindung betrifft ein neues, von Pivaloylcyanid ausgehendes Verfahren zur Herstellung des bekannten, herbizid wirksamen 4-Amino-6-tert.-butyl-3-methylthio-1,2,4-triazin-5(4H)-ons.

Es sind bereits Verfahren zur Herstellung von 4-Amino-6-tert.-butyl-3-methylthio-1,2,4-triazin-5(4H)-on (I), die von Pivaloylcyanid oder anderen Pivalinsäurederivaten ausgehen, bekanntgeworden. Sie unterscheiden sich in der Methode der Herstellung des Zwischenproduktes 4-Amino-6-tert.-butyl-3-mercapto-1,2,4-triazin-5(4H)-on (VI).

Nach DE-OS 21 65 554 kann man das Triazinon (I) herstellen durch Umsetzung von Pivaloylchlorid mit einem Isonitril, Hydrolysieren des gebildeten Imidchlorids zum entsprechenden Trimethylbrenztraubensäureamid, Weiterumsetzung des Amids mit Thiocarbohydrazid und Methylierung des erhaltenen Cyclisierungsproduktes (VI) (Ausbeuten: 60-82% d. Th. an (VI); 49-67% an (I), jeweils bezogen auf Pivaloylchlorid).

Nach DE-OS 22 21 771 ist es möglich, das genannte Triazinon (I) herzustellen, indem man ein Pivalinsäureamid, z.B. Pivalanilid, durch Chlorierung, z.B. mittels Thionylchlorid, in das entsprechende Pivalimidochlorid überführt, dieses mit einem Metallcyanid, beispielsweise Kupfer (I)-cyanid, oder Cyanwasserstoff gegebenenfalls in Gegenwart eines Katalysators zu dem entsprechenden α-Imino-nitril umsetzt, letzteres durch Umsetzung mit Thiocarbohydrazid zu 4-Amino-6-tert.-butyl-5-imino-3-mercapto-1,2,4-triazin cyclisiert, dann die 5-Iminogruppe zur 5-Ketogruppe hydrolysiert, wobei man das Zwischenprodukt (VI) erhält, und dieses anschliessend methyliert (Ausbeuten: 42-57% d. Th. an (VI); 35-47% d. Th. an (I), jeweils bezogen auf Pivalanilid).

Beide Verfahren sind technisch ausserordentlich aufwendig, verlaufen mit unbefriedigenden Ausbeuten und sind damit zur Übertragung in den grosstechnischen Massstab ungeeignet.

Gemäss DE-OS 27 33 180 kann das genannte Triazinon (I) hergestellt werden, indem man Pivaloylcyanid in einer sogenannten Ritter-Reaktion mit t-Butanol oder Isobutylen zu Trimethylbrenztraubensäure-N-t-butylamid umsetzt und dieses, gegebenenfalls nach vorheriger Verseifung zur freien Trimethylbrenztraubensäure, mit Thiocarbohydrazid zu dem Zwischenprodukt (VI) cyclisiert und letzteres anschliessend methyliert (Ausbeuten: 51-67% d. Th. an (VI); 41-54% an (I), jeweils bezogen auf Pivaloylcyanid).

Das letztgenannte Verfahren hat den grundsätzlichen Nachteil, dass sich das als Zwischenprodukt auftretende Trimethylbrenztraubensäure-N-t-butylamid nur relativ schwer weiter umsetzen lässt; dies gilt sowohl für die Hydrolyse zur freien Ketosäure wie auch für die Ringschlussreaktion mit Thiocarbohydrazid.

So gelingt die saure Hydrolyse des Trimethylbrenztraubensäure-N-t-butylamids zu Trimethylbrenztraubensäure durch 10stündiges Erhitzen unter Rückfluss in 5w-HCl und nachfolgende extraktive Aufarbeitung mit $CH_2Cl_2$, verdünnter wässeriger NaOH-Lösung, konzentrierter Salzsäure und Essigester lediglich in einer Ausbeute von 75% d. Th.

Verzichtet man auf eine vorausgehende Hydrolyse des α-Ketocarbonsäure-N-t-butylamids und setzt dieses direkt mit Thiocarbohydrazid um, so kann das Cyclisierungsprodukt 4-Amino-6-tert.-butyl-3-mercapto-1,2,4-triazin-5(4H)-on nach mehrstündigem Erhitzen (bis zu 8 Stunden) unter Rückfluss lediglich in Ausbeuten von 72% d. Th. isoliert werden. Wie eigene Versuche darüber hinaus gezeigt haben, verläuft die Reaktion von α-Ketocarbonsäure-N-alkylamiden mit Thiocarbohydrazid nach DE-OS 21 65 554 und DE-OS 27 33 180 nicht einheitlich zu 4-Amino-6-tert.-butyl-3-mercapto-1,2,4-triazin-5(4H)-on, sondern unter Bildung zahlreicher Nebenprodukte.

Es wurde nun gefunden, dass man 4-Amino-6-tert.-butyl-3-methylthio-1,2,4-triazin-5(4H)-on der Formel I

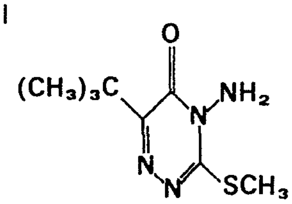

(I)

ausgehend von Pivaloylcyanid in überraschend einfacher Weise mit hoher Ausbeute und in hoher Reinheit erhält, wenn man in einer ersten Stufe Pivaloylcyanid der Formel II

$$(CH_3)_3C-CO-CN \quad (II)$$

mit einem Carbonsäureanhydrid der allgemeinen Formel III

$$R-CO-O-CO-R \quad (III)$$

in welcher

R für gegebenenfalls chlorsubstituiertes Alkyl mit 1 bis 4 Kohlenstoffatomen oder für Phenyl steht,

in Gegenwart einer starken Säure, ausgewählt aus der Gruppe bestehend aus anorganischen Sauerstoffsäuren, Lewis-Säuren, aliphatischen und aromatischen Sulfon- und Phosphonsäuren sowie Halogenalkancarbonsäuren, und gegebenenfalls in Gegenwart eines Losungsmittels bei Temperaturen zwischen −50 und +150°C umsetzt und das Reaktionsgemisch anschliessend mit Wasser versetzt, in einer zweiten Stufe die hierbei gebildeten, bisher nicht bekannten Trimethylbrenztraubensäure-N-acylamide der allgemeinen Formel IV

$$(CH_3)_3C-CO-CO-NH-CO-R \quad (IV)$$

in welcher

R die oben angegebene Bedeutung hat,

gegebenenfalls nach vorheriger Verseifung zur freien Trimethylbrenztraubensäure der Formel V

$$(CH_3)_3C-CO-COOH \qquad (V)$$

mit Thiocarbohydrazid ($NH_2-NH-CS-NH-NH_2$) bei Temperaturen von −20 bis +150°C zu 4-Amino-6-tert.-butyl-3-mercapto-1,2,4-triazin-5-(4H)-on der Formel VI

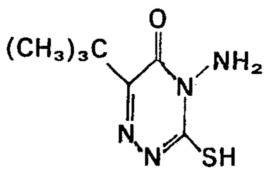

(VI)

umsetzt und in einer dritten Stufe dieses Zwischenprodukt (VI) in üblicher Weise methyliert.

Die erste Stufe des erfindungsgemässen Verfahrens beinhaltet eine gegenüber dem Stand der Technik neuartige und vorteilhafte Verfahrensweise, wobei in überraschend glattem und einheitlichem Reaktionsverlauf die neuen Trimethylbrenztraubensäure-N-acylamide (IV) gebildet werden.

Während die Herstellung von N-Acylamiden durch Umsetzung von Nitrilen mit Carbonsäuren bzw. deren Anhydriden in Gegenwart von Katalysatoren, wie z.B. Mineralsäuren, für eine Reihe aliphatischer und aromatischer Nitrile beschrieben ist (vgl. Compr. Org. Chem. *2*, S. 539 (1979)), ist die entsprechende Umwandlung in der Klasse der Acylcyanide zu α-Ketocarbonsäure-N-acylamiden bisher nicht bekannt.

Die Umsetzung der erfindungsgemäss gut zugänglichen Trimethylbrenztraubensäure-N-acylamide (IV) mit Thiocarbohydrazid gelingt bereits unter milden Bedingungen und führt in hoher Ausbeute zu sehr reinem 4-Amino-6-tert.-butyl-3-mercapto-1,2,4-triazin-5(4H)-on (VI).

Das erfindungsgemässe Verfahren vermeidet die zuvor erwähnten, mit den vergleichbaren vorbekannten Verfahren zur Herstellung des herbiziden Wirkstoffes (I) verbundenen Nachteile; dies bedeutet eine wesentliche technische Vereinfachung.

Gegenüber anderen vorbekannten Verfahren (vgl. z.B. DE-OS'en 20 03 144, 24 60 889, 24 60 909, 26 48 300) zur Herstellung des Wirkstoffes (I) aus anderen Pivalinsäurederivaten bzw. Pinakolin hat das erfindungsgemässe Verfahren ebenfalls den technischen Vorteil starker Vereinfachung. Gegenüber den von Pinakolin ausgehenden Verfahren ist ein zusätzlicher Vorteil in der unterschiedlichen Rohstoffbasis zu sehen. Verwendet man in der ersten Verfahrensstufe Essigsäureanhydrid als Carbonsäureanhydrid der allgemeinen Formel (III), und konzentrierte Schwefelsäure als starke Säure, setzt in der zweiten Stufe das N-Acetylamid-Zwischenprodukt (IVa) als solches mit Thiocarbohydrazid um und verwendet in der dritten Stufe Methylbromid als Methylierungsmittel, so kann der Reaktionsverlauf nach dem erfindungsgemässen Verfahren zusammenfassend nochmals durch das folgende Formelschema wiedergegeben werden:

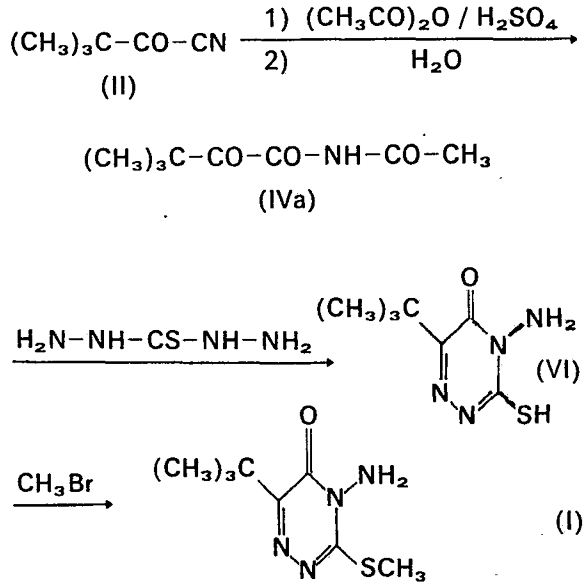


Das als Ausgangsprodukt verwendete Pivaloylcyanid (II) ist bekannt und kann z.B. durch Umsetzung von Pivaloylchlorid mit Kupfer-(I)-cyanid hergestellt werden (vgl. z.B. J. Amer. Chem. Soc. *72*, S. 2793 (1950)).

Die weiterhin als Ausgangsstoffe einzusetzenden Carbonsäureanhydride sind durch Formel (III) allgemein definiert. Sie sind zum Teil grosstechnisch verfügbar bzw. nach allgemein bekannten Methoden z.B. aus den entsprechenden Carbonsäuren herstellbar.

Im Rahmen dieser Erfindung besonders bevorzugte Carbonsäureanhydride sind Essigsäureanhydrid, Propionsäureanhydrid und die Anhydride der Chloressigsäuren.

Zu Stufe 1 des erfindungsgemässen Verfahrens seien im einzelnen noch folgende Angaben gemacht:

Die erste Verfahrensstufe wird in Gegenwart einer starken Säure durchgeführt. Als solche Säuren kommen anorganische Sauerstoffsäuren, wie konzentrierte Schwefelsäure, sowie Perchlorsäure, Salpetersäure und Phosphorsäure, ferner Lewis-Säuren wie Bortrifluorid, Aluminiumchlorid oder Zinkchlorid in Frage. Weiterhin geeignet sind aliphatische und aromatische Sulfon- und Phosphonsäuren sowie Halogenalkancarbonsäuren wie z.B. Trichloressigsäure. Es ist auch möglich, die Umsetzung in Gegenwart mehrerer solcher Säuren durchzuführen. Bevorzugt werden anorganische Sauerstoffsäuren, insbesondere konzentrierte Schwefelsäure, verwendet.

Die Reaktionstemperaturen können bei dieser Verfahrensstufe in einem grösseren Bereich variiert werden. Im allgemeinen arbeitet man, wie oben angegeben, bei Temperaturen zwischen etwa −50 und +150°C, vorzugsweise zwischen etwa 0 und 100°C. Die anschliessende Aufarbeitung wird zweckmässig mittels Eiswasser durchgeführt.

Die Reaktion wird im allgemeinen bei Normaldruck durchgeführt.

Die Umsetzung gemäss Verfahrensstufe 1 kann in Abwesenheit oder in Gegenwart eines Lösungsmittels bzw. Lösungsvermittlers durchgeführt werden. Als Lösungsvermittler kommen bestimmte organische Lösungsmittel in Frage; besonders geeignet sind Eisessig und Dichlormethan, ferner Dialkyläther, wie Diäthyl- oder Diisopropyläther, und Diaryläther, wie z.B. Diphenyläther.

Bei der Durchführung der ersten Stufe des erfindungsgemässen Verfahrens setzt man im allgemeinen auf 1 Mol Pivaloylcyanid der Formel (II) 0,5 bis 10 Mol, vorzugsweise 0,8 bis 4 Mol Carbonsäureanhydrid der Formel (III) ein; besonders bevorzugt ist ein Molverhältnis von Pivaloylcyanid (II) zu Carbonsäureanhydrid (III) von 1:1 bis 1:2.

Die für die Durchführung der ersten Stufe des erfindungsgemässen Verfahrens erforderlichen Säuren werden in katalytischen bis überstöchiometrischen Mengen eingesetzt. Im allgemeinen setzt man auf 1 Mol Pivaloylcyanid (II) 0,5 bis 10 Mol, vorzugsweise 0,8 bis 8 Mol, besonders bevorzugt 1 bis 4 Mol Säure ein.

Besonders vorteilhaft ist ein Molverhältnis Carbonsäureanhydrid (III) zu Säure von 1:2.

Damit ergibt sich, dass ein Molverhältnis Pivaloylcyanid (II) zu Carbonsäureanhydrid (III) zu Säure von 1:1:2 bis 1:2:4 ganz besonders günstig ist.

Zweckmässigerweise geht man bei der Durchführung der ersten Stufe des Verfahrens so vor, dass man die Säure und Carbonsäureanhydrid (III) bzw. ein Gemisch aus Lösungsmittel, Säure und Carbonsäureanhydrid (III) vorlegt und Pivaloylcyanid (II), gegebenenfalls in Lösungsmittel, zugibt.

Die Reaktionszeiten betragen im allgemeinen 1 bis 10 Stunden. Das Reaktionsgemisch wird anschliessend am zweckmässigsten auf Eis gegossen. Die gebildeten Trimethylbrenztraubensäure-N-acylamide können durch Filtration oder durch Extraktion isoliert werden.

Hierzu geeignete Extraktionsmittel sind mit Wasser nicht beliebig mischbare Lösungsmittel, beispielsweise Äther wie Diethyläther oder Diisopropyläther, Ester wie z.B. Essigsäureäthylester, Ketone wie z.B. Methylisobutylketon, Halogenkohlenwasserstoffe wie z.B. Dichlormethan, Chlorbenzol oder Dichlorbenzol, ferner Aromaten wie z.B. Benzol, Toluol, o-Xylol, Äthylbenzol, Cumol oder Nitrobenzol. Vorzugsweise verwendet man Dichlormethan.

Die in der ersten Verfahrensstufe gebildeten Trimethylbrenztraubensäure-N-acylamide (IV) können, wenn gewünscht, durch saure Hydrolyse leicht zur freien Trimethylbrenztraubensäure-(3,3-Dimethyl-1-oxo-buttersäure) (V) verseift werden.

Die Umsetzung der freien Trimethylbrenztraubensäure (V) mit Thiocarbohydrazid zum Cyclisierungsprodukt (VI) ist bekannt (vgl. z.B. DE-OS 24 60 889).

In der zweiten Stufe des erfindungsgemässen Verfahrens werden jedoch vorzugsweise die neuen Trimethylbrenztraubensäure-N-acylamide (IV) direkt, d.h. ohne vorherige Verseifung derselben zur freien Säure (V), mit Thiocarbohydrazid zu (VI) umgesetzt. Diese Umsetzung wird beispielsweise in einer mineralsauren, vorzugsweise einer salzsauren, wässerigen bzw. einer mineralsauren, wässerig-alkoholischen Lösung durchgeführt.

Die Reaktionstemperaturen können bei dieser Verfahrensstufe in einem grösseren Bereich variiert werden. Im allgemeinen arbeitet man zwischen −20 und +150°C, vorzugsweise zwischen 0 und 100°C.

Bei der Durchführung der zweiten Verfahrensstufe setzt man die Ausgangsstoffe vorzugsweise in molaren Mengen ein. Die Isolierung des Zwischenproduktes (VI) erfolgt in üblicher Weise.

Das Zwischenprodukt (VI) kann auch in Form des tautomeren 4-Amino-6-tert.-butyl-5-oxo-3-thioxo-tetrahydro-1,2,4(2H,4H)-triazins der Formel

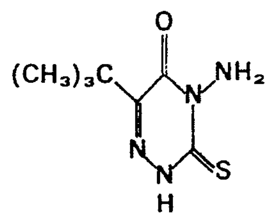

(VIa)

vorliegen; der Einfachheit wegen wird hier jedoch für beide Tautomeren (VI) bzw. (VIa) stets die Bezeichnung 4-Amino-6-tert.-butyl-3-mercapto-1,2,4-triazin-5(4H)-on (VI) verwendet.

Die dritte Stufe des erfindungsgemässen Verfahrens erfolgt in bekannter Weise durch Umsetzung von (VI) mit einem Methylierungsmittel, wie z.B. Methylbromid oder Methyljodid, in Gegenwart einer Base, wie Natriumhydroxid, in wässeriger Lösung bei Temperaturen zwischen 0 und 50°C.

Das erfindungsgemäss herstellbare 4-Amino-6-tert.-butyl-3-methylthio-1,2,4-triazin-5(4H)-on der Formel (I) (Metribuzin) zeichnet sich bekanntermassen durch hervorragende herbizide Wirksamkeit aus (vgl. z.B. DE-PS 17 95 784).

Die nachfolgenden Herstellungsbeispiele sollen das erfindungsgemässe Verfahren näher illustrieren.

*Herstellungsbeispiele*

*Beispiel 1*

a) *Stufe 1:*

$(CH_3)_3C-CO-CO-NH-CO-CH_3$ (IVa)

In 49,0 g (0,5 Mol) vorgelegte konzentrierte Schwefelsäure werden jeweils bei Raumtemperatur zunächst 25,6 g (0,25 Mol) Essigsäureanhydrid und anschliessend 27,8 g (0,25 Mol) Pivaloylcyanid eingetragen. Nach 4stündigem Nachrühren wird die Reaktionsmischung mit 150 g Eiswasser versetzt und gut durchgerührt. Das ausfallende Reaktionsprodukt wird abgesaugt, mit 100 ml Wasser gewaschen und getrocknet. Man erhält 37,0 g (86,5% d. Th.) Trimethylbrenztraubensäure-N-acetylamid als farblose glänzende Blättchen vom Schmelzpunkt 82-84°C; Gehalt nach gaschromatographischer Bestimmung >98%. Für weitere Umsetzungen sind keine zusätzlichen Reinigungsoperationen erforderlich.

b) *Stufe 2:* 4-Amino-6-tert.-butyl-3-mercapto-1,2,4-triazin-5(4H)-on (VI)

Zu 23,2 g (0,22 Mol) Thiocarbohydrazid in 400 ml 1n-HCl lässt man 37,0 g (0,22 Mol) Trimethylbrenztraubensäure-N-acetylamid in 150 ml Äthanol zutropfen und lässt die Reaktionsmischung 5 Stunden lang bei Raumtemperatur nachrühren. Das ausgefallene Produkt wird abgesaugt, mit Wasser gewaschen und getrocknet. Man erhält 41,2 g des oben bezeichneten Produktes (VI) vom Schmelzpunkt 210°C, mit einem gaschromatographisch bestimmten Gehalt von > 99%, was einer Ausbeute von 95% d. Th. entspricht.

c) *Stufe 3:* 4-Amino-6-tert.-butyl-3-methylthio-1,2,4-triazin-5(4H)-on (I)

In eine Mischung aus 200 g 45%iger Natronlauge und 135 g Wasser werden unter Rühren 41,2 g (0,206 Mol) 4-Amino-6-tert.-butyl-3-mercapto-1,2,4-triazin-5(4H)-on (VI) eingetragen. Nach vollständiger Lösung des Produktes werden 34,0 g Methyljodid so zugegeben, dass die Innentemperatur 30°C nicht übersteigt. Nach beendeter Zugabe wird das Reaktionsgemisch noch 2 Stunden bei Raumtemperatur gerührt. Dann wird das ausgefallene Reaktionsprodukt abgesaugt, mit 200 ml Wasser gewaschen und getrocknet. Man erhält 35,6 g (81% d. Th.) 4-Amino-6-tert.-butyl-3-methylthio-1,2,4-triazin-5(4H)-on (I) vom Schmelzpunkt 123-125°C.

Für die grosstechnische Durchführung der Methylierung gemäss Stufe 3 stehen mehrere verbesserte Verfahren zur Verfügung (vgl. z.B. DE-OS 27 29 761, ferner US-PS' en 3 890 317, 3 897 429, 3 905 973, 4 035 364).

*Beispiel 2*

a) Hydrolyse (IVa) $\xrightarrow{H_2O / H^+}$

$(CH_3)_3C-CO-COOH$ (V)

17,1 g (0,1 Mol) Trimethylbrenztraubensäure-N-acetylamid (IVa) werden in 100 ml 5n-HCl 4 Stunden auf 90°C erhitzt. Nach dem Abkühlen schüttelt man mit Methylenchlorid aus, extrahiert die Methylenchloridphase mit verdünnter NaOH-Lösung, stellt die alkalische wässerige Lösung mit konzentrierter HCl auf pH 1, schüttelt mit Essigester aus und dampft anschliessend den Essigesterextrakt ein. Man erhält 11,9 g (92% d. Th.) Trimethylbrenztraubensäure (V).

b) Die Umwandlung von Trimethylbrenztraubensäure in die Triazinone (IV) und (I) ist bekannt (vgl. z.B. DE-OS' en 24 60 889 und 27 33 180).

**Patentansprüche:**

1. Verfahren zur Herstellung von 4-Amino-6-tert.-butyl-3-methylthio-1,2,4-triazin-5(4H)-on der Formel I

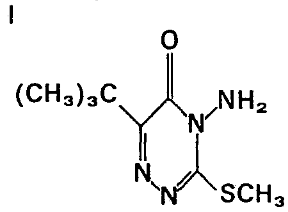

(I)

dadurch gekennzeichnet, dass man
(1) Pivaloylcyanid der Formel II

$$(CH_3)_3C-CO-CN \quad (II)$$

mit einem Carbonsäureanhydrid der allgemeinen Formel III

$$R-CO-O-CO-R \quad (III)$$

in welcher

R für gegebenenfalls chlorsubstituiertes Alkyl mit 1 bis 4 Kohlenstoffatomen oder für Phenyl steht, in Gegenwart einer starken Säure, ausgewählt aus der Gruppe bestehend aus anorganischen Sauerstoffsäuren, Lewis-Säuren, aliphatischen und aromatischen Sulfon- und Phosphonsäuren sowie Halogenalkancarbonsäuren, und gegebenenfalls in Gegenwart eines Lösungsmittels bei Temperaturen zwischen −50 und +150°C umsetzt und das Reaktionsgemisch anschliessend mit Wasser versetzt,

(2) die hierbei gebildeten neuen Trimethylbrenztraubensäure-N-acylamide der allgemeinen Formel IV

$$(CH_3)_3C-CO-CO-NH-CO-R \quad (IV)$$

in welcher

R die oben angegebene Bedeutung hat, gegebenenfalls nach vorheriger Verseifung zur freien Trimethylbrenztraubensäure der Formel V

$$(CH_3)_3C-CO-COOH \quad (V)$$

mit Thiocarbohydrazid ($NH_2-NH-CS-NH-NH_2$) bei Temperaturen zwischen −20 und +150°C zu 4-Amino-6-tert.-butyl-3-mercapto-1,2,4-triazin-5(4H)-on der Formel VI

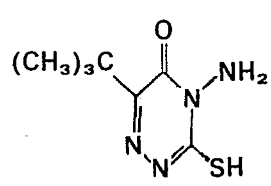

(VI)

umsetzt und

(3) dieses Zwischenprodukt (VI) in üblicher Weise methyliert.

2. Verfahren nach Anspruch 1, dadurch gekennzeichnet, dass man die Umsetzung gemäss Stufe (1) bei Temperaturen zwischen 0 und 100°C durchführt.

3. Verfahren nach Anspruch 1, dadurch gekennzeichnet, dass man bei der Umsetzung gemäss Stufe (1) Pivaloylcyanid (II) und Carbon-

säureanhydrid (III) im Molverhältnis von 1:0,5-10 einsetzt.

4. Verfahren nach Anspruch 1, dadurch gekennzeichnet, dass man bei der Umsetzung gemäss Stufe (1) Pivaloylcyanid (II) und Säure im Molverhältnis von 1:0,5-10 einsetzt.

5. Verfahren nach Anspruch 1, dadurch gekennzeichnet, dass man bei der Umsetzung gemäss Stufe (1) Carbonsäureanhydrid und Säure im Molverhältnis 1:2 einsetzt.

6. Verfahren nach Anspruch 1, dadurch gekennzeichnet, dass man bei der Umsetzung gemäss Stufe (1) Pivaloylcyanid (II), Carbonsäureanhydrid (III) und Säure im Molverhältnis von 1:1:2 bis 1:2:4 einsetzt.

7. Verfahren nach Anspruch 1, dadurch gekennzeichnet, dass man bei der Umsetzung gemäss Stufe (1) als Carbonsäureanhydrid der Formel (III) Essigsäureanhydrid einsetzt und dass die Umsetzung gemäss Stufe (2) mit Trimethylbrenztraubensäure-N-acetylamid (IVa) durchgeführt wird.

8. Verfahren nach Anspruch 1, dadurch gekennzeichnet, dass man bei der Umsetzung gemäss Stufe (1) als starke Säure konzentrierte Schwefelsäure einsetzt.

9. Verfahren zur Herstellung von 4-Amino-6-tert.-butyl-3-mercapto-1,2,4-triazin-5(4H)-on (VI) nach Stufen (1) und (2) des Verfahrens gemäss Anspruch 1.

**Revendications:**

1. Procédé de production de 4-amino-6-tert.-butyl-3-méthylthio-1,2,4-triazine-5-(4H)-one de formule (I):

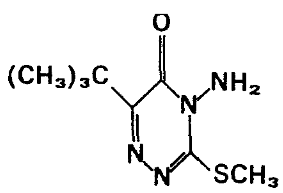

(I)

caractérisé en ce que:

1) on fait réagir le cyanure de pivaloyle de formule (II):

$$(CH_3)_3C-CO-CN \quad (II)$$

avec un anhydride d'acide carboxylique de formule générale (III):

$$R-CO-O-CO-R \quad (III)$$

dans laquelle R désigne un groupe alkyle ayant 1 à 4 atomes de carbone, éventuellement substitué par du chlore, ou le groupe phényle, en présence d'un acide fort choisi dans le groupe formé d'oxacides inorganiques, d'acides de Lewis, d'acides sulfoniques et phosphoniques aliphatiques et aromatiques ainsi que d'acides halogénoalcanecarboxyliques, le cas échéant, en présence d'un solvant, à des températures comprises entre −50 et +150°C, et on ajoute ensuite de l'eau au mélange réactionnel,

2) on fait réagir les nouveaux N-acylamides d'acide triméthylpyruvique formés à cette occasion, de formule générale (IV):

$$(CH_3)_3C-CO-CO-NH-CO-R \quad (IV)$$

dans laquelle R a la définition indiquée ci-dessus, le cas échéant après saponification préalable en acide triméthylpyruvique libre de formule (V):

$$(CH_3)_3C-CO-COOH \quad (V)$$

avec le thiocarbohydrazide ($NH_2-NH-CS-NH-NH_2$), à des températures comprises entre −20 et +150°C, pour obtenir la 4-amino-6-tert.-butyl-3-mercapto-1,2,4-triazine-5-(4H)-one de formule (VI):

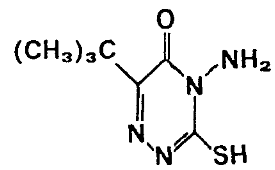

(VI)

3) on méthyle de façon classique ce produit intermédiaire (VI).

2. Procédé suivant la revendication 1, caractérisé en ce qu'on conduit la réaction suivant l'étape 1 à des températures comprises entre 0 et 100°C.

3. Procédé suivant la revendication 1, caractérisé en ce qu'on utilise dans la réaction suivant l'étape 1 le cyanure de pivaloyle (II) et l'anhydride d'acide carboxylique (III) dans le rapport molaire de 1:0,5-10.

4. Procédé suivant la revendication 1, caractérisé en ce qu'on utilise dans la réaction suivant l'étape 1 le cyanure de pivaloyle (II) et l'acide dans un rapport molaire de 1:0,5-10.

5. Procédé suivant la revendication 1, caractérisé en ce qu'on utilise dans la réaction selon l'étape 1 l'anhydride d'acide carboxylique et l'acide dans le rapport molaire de 1:2.

6. Procédé suivant la revendication 1, caractérisé en ce qu'on utilise dans la réaction selon l'étape 1 le cyanure de pivaloyle (II), l'anhydride d'acide carboxylique (III) et l'acide dans la proportion molaire de 1:1:2 à 1:2:4.

7. Procédé suivant la revendication 1, caractérisé en ce qu'on utilise dans la réaction selon l'étape 1 l'anhydride d'acide acétique comme anhydride d'acide carboxylique de formule (III) et en ce qu'on conduit la réaction selon l'étape 2 avec le N-acétylamide d'acide triméthylpyruvique (IVa).

8. Procédé suivant la revendication 1, caractérisé en ce qu'on utilise l'acide sulfurique concentré comme acide fort dans la réaction selon l'étape 1.

9. Procédé de production de la 4-amino-6-tert.-butyl-3-mercapto-1,2,4-triazine-5-(4H)-one (VI) suivant les étapes 1 et 2 du procédé conforme à la revendication 1.

**Claims:**

1. Process for the preparation of 4-amino-6-tert.-butyl-3-methylthio-1,2,4-triazin-5-(4H)-one of the formula (I):

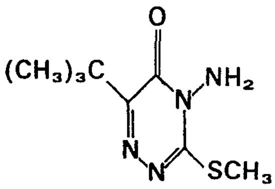

(I)

characterized in that: (1) pivaloyl cyanide of the formula (II):

$$(CH_3)_3C-CO-CN \quad (II)$$

is reacted with a carboxylic acid anhydride of the general formula (III):

$$R-CO-O-CO-R \quad (III)$$

in which R represents optionally chlorine-substituted alkyl with 1 to 4 carbon atoms or phenyl, in the presence of a strong acid selected from the group consisting of inorganic oxyacids, Lewis acids, aliphatic and aromatic sulphonic and phosphonic acids and halogenoalkanecarboxylic acids, and if appropriate in the presence of a solvent, at temperatures between −50 and +150°C, and water is then added to the reaction mixture,

(2) the new trimethylpyruvic acid N-acylamides thereby formed, of the general formula (IV):

$$(CH_3)_3C-CO-CO-NH-CO-R \quad (IV)$$

in which R has the abovementioned meaning, are reacted, if appropriate after prior hydrolysis to the free trimethylpyruvic acid of the formula (V):

$$(CH_3)_3C-CO-COOH \quad (V)$$

with thiocarbohydrazide ($NH_2-NH-CS-NH-NH_2$), at temperatures between −20 and +150°C, to give 4-amino-6-tert.-butyl-3-mercapto-1,2,4-triazin-5-(4H)-one of the formula (VI):

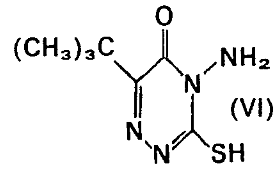

(VI)

(3) this intermediate product (VI) is methylated in the customary manner.

2. Process according to claim 1, characterized in that the reaction in stage 1 is carried out at temperatures between 0 and 100°C.

3. Process according to claim 1, characterized in that, in the reaction in stage 1, pivaloyl cyanide (II) and carboxylic acid anhydride (III) are employed in a molar ratio of 1:0.5-10.

4. Process according to claim 1, characterized in that, in the reaction in stage 1, pivaloyl cyanide (II) and the acid are employed in a molar ratio of 1:0.5-10.

5. Process according to claim 1, characterized in that, in the reaction in stage 1, carboxylic acid anhydride and the acid are employed in a molar ratio of 1:2.

6. Process according to claim 1, characterized in that, in the reaction in stage 1, pivaloyl cyanide (II), carboxylic acid anhydride (III) and the acid are employed in a molar ratio of 1:1:2 to 1:2:4.

7. Process according to claim 1, characterized in that, in the reaction in stage 1, acetic anhydride is employed as the carboxylic acid anhydride of the formula (III), and in that the reaction in stage 2 is carried out with trimethylpyruvic acid N-acetylamide (IVa).

8. Process according to claim 1, characterized in that, in the reaction in stage 1, concentrated sulphuric acid is employed as the strong acid.

9. Process for the preparation of 4-amino-6-tert.-butyl-3-mercapto-1,2,4-triazin-5-(4H)-one (VI) by stages 1 and 2 of the process according to claim 1.